# EUROPEAN PATENT APPLICATION

(11) **EP 1 952 812 A1**
(43) Date of publication of application: **06.08.2008**
(21) Application number: 08100891.4
(22) Date of filing: 24.01.2008
(51) Int. Cl.: A61K 31/37, A61K 41/00, A61P 7/06

(54) **Drug for treating thalessemia, falciform anemia and all other forms of anemia treatable therewith**

(30) Priority: 25.01.2007 IT VE20070005
(71) Applicant: Universita' Degli Studi di Ferrara, 44100 Ferrara (IT); Universita' Degli Studi Di Padova, 35122 Padova (IT); Associazione Veneta per la Lotta alla Talassemia, 45100 Rovigo (IT)
(72) Inventor: Bianchi, Nicoletta, 44100 Ferrara (IT); Borgatti, Monica, 44100, Ferrara (IT); Gambari, Roberto, 44100, Ferrara (IT); Lampronti, Ilaria, 44100, Ferrara (IT); Dall'Acqua, Francesco, 35122, Padova (IT); Vedaldi, Daniela, 35122, Padova (IT); Viola, Giampietro, 35122, Padova (IT)
(74) Representative: Piovesana, Paolo

(57) **Abstract**

A drug for treating thalessemia, falciform anemia and all other forms of anemia treatable therewith, characterised by comprising as active principle a furocoumarin or a heteroanalogue thereof in a quantity unsuitable for providing any significant therapeutic effect in the fundamental state, to be activated with UVA light after its administration.

## Description

The present invention relates to a drug for treating thalessemia, falciform anemia and all other forms of anemia treatable therewith.

The treatment is based on effectively inducing erythroid cell differentiation and gamma-globin messenger RNA production increase associated with increased biosynthesis of fetal hemoglobin (HbF).

The existence of substances able to induce gamma-globin gene expression and hemoglobin HbF synthesis in adult subjects has been known for some time (Rodgers GP, Rachmilewitz EA, British J. Haematology, 91:263-268, 1995; Rochette J, Craig JE and Thein SL, Blood Reviews, 8: 213-224, 1994; Dover GJ, Brusilow S, Samid D, New England Journal of Medicine, 327: 569-570, 1992; Ikuta T, Atweh G, Boosalis V, White GL, De Fonseca S, Boosalis M, Faller DV, Perrine SP, Annals of New York Academy of Sciences, 850:87-99, 1998). In most cases, these substances are also able to activate or potentiate gene transcription for embryonal and fetal globins in experimental models.

In the human being, activating gene transcription for gamma-globins in adult subjects leads to the production of fetal hemoglobin by miming the phenotype (HPFH) (high persistence of fetal hemoglobin) which confers favourable clinical symptoms on patients affected by beta-thalessemia, including in homozygot form (Ikuta T, Atweh G, Boosalis V, White GL, De Fonseca S, Boosalis M, Faller DV, Perrine SP, Annals of New York Academy of Sciences, 850:87-99, 1998).

It is also known that angelicin - a natural and synthetic isopsoralen derivative in the fundamental state - and its structural analogues, again in the fundamental state, are able to induce said gamma-globin gene expression and the biosynthesis of HbK (Lampronti I., Bianchi N Borgatti M., Fibach E.,Prus E., Gambari R. Eur J Haematology 71 189-195 2003). Because of this activity, these molecules can be of interest in the therapy of beta-thalessemia and falciform anemia (patent application TO2002A000684 of 31 July 2002). This activity is unexpected in the light of the known therapeutic uses for angelicin and its structural analogues (Kong LD, Tan RX, Woo AY, Cheng CH. Pharmacol Toxicol, 88(2):75-80, 2001; Mosti L, Lo Presti E, Menozzi G, Marzano C, Baccichetti F, Falcone G, Filippelli W, Piucci B. II Farmaco, 53(8-9):602-10, 1998; Sardari S, Mori Y, Horita K, Micetich RG, Nishibe S, Daneshtalab M. Bioorg Med Chem, 7(9):1933-40, 1999; Jakobs AE, Christiaens L. J Org Chem, 61(14):4842-4844, 1996; lester M, Fossa P, Menozzi G, Mosti L, Baccichetti F, Marzano C, Simonato M. II Farmaco, 50 (10): 669-678, 1995; Bisagni E. Photochem Photobiol, Review, 14(1-2):23-46, 1992; Dall'Acqua F, Vedaldi D, Bordin F, Baccichetti F, Carlassare F, Tamaro M, Rodighiero P, Pastorini G, Guiotto A, Recchia G, Cristofolini. J Med Chem, 26(6):870-6, 1983; Dall'Acqua F, Vedaldi D, Guiotto A, Rodighiero P, Carlassare F, Baccichetti F, Bordin F. J Med Chem, 24(7):806-11, 1981; Conconi MT, Montesi F, Parnigotto PP. Pharmacol Toxicol, 82(4):193-8, 1998; Marzano C, Severin E, Pani B, Guiotto A, Bordin F. Environ Mol Mutagen, 29(3):256-64, 1997; Bordin F, Dall'Acqua F, Guiotto A. Pharmacol Ther. Review, 52(3):331-63, 1991; Backhouse CN, Delporte CL, Negrete RE, Erazo S, Zuniga A, Pinto A, Cassels BK. J. Ethnopharmacol, 78(1): 27-31, 2001).

Angelicin, in its fundamental state, has in fact been proposed in the literature as an antiinflammatory agent (Backhouse CN, Delporte CL, Negrete RE, Erazo S, Zuniga A, Pinto A, Cassels BK. J. Ethnopharmacol, 78(1): 27-31, 2001) and antifungal (Sardari S, Mori Y, Horita K, Micetich RG, Nishibe S, Daneshtalab M. Bioorg Med Chem, 7(9):1933-40, 1999).

Its structural formula is the following:

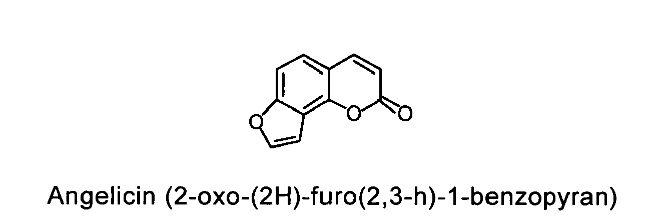

Moreover, angelicin and some of its structural analogues, when photoactivated with UVA light, have been proposed in the literature for treating psorfiasis (PUVA therapy) (Furocoumarin for the photochemotherapy of psoriasis and related skin diseases. U.S.A. patent 4312883 and U.S.A. patent 5001147 Dall'Acqua F, Vedaldi D, Bordin F, Baccichetti F, Carlassare F, Tamaro M, Rodighiero P, Pastorini G, Guiotto A, Recchia G, Cristofolini. J Med Chem, 26(6):870-6, 1983; Dall'Acqua F, Vedaldi D, Guiotto A, Rodighiero P, Carlassare F, Baccichetti F, Bordin F. J Med Chem, 24(7):806-11, 1981), fungoid mycosis and other skin diseases, as antiproliferative ed immunomodulator agents (Bordin F, Dall'Acqua F, Guiotto A. Pharmacol Ther. Review, 52(3):331-63, 1991; Backhouse CN, Delporte CL, Negrete RE, Erazo S, Zuniga A, Pinto A, Cassels BK. J. Ethnopharmacol, 78(1): 27-31, 2001; F. Dall'Acqua, G.Viola, D. Vedaldi Molecular basis of psoralen photochemotherapy (Chapter 142) in CRC Handbook of Organic Photochemistry and Photobiology. W.M. Hoorspool and F. Lenci Eds. CRC Press Boca Raton USA 2004, pp. 1-17). In the case of PUVA therapy, the patient assumes furocoumarin orally or by topical application and is then subjected to skin irradiation with UVA light. Furocoumarins have also been proposed as pigmentation inducers by stimulating the protein kinase C (PKC) and consequently the tyrosinase enzyme (Bordin F, Dall'Acqua F, Guiotto A. Pharmacol Ther, Review, 52(3):331-63, 1991; Caffieri S., Ruzzene M., Guerra B., Frank S.,Vedaldi D., Dall'Acqua F. J. Photochem.Photobiol. B: Biol. 22, 253-257 1994; Anthony F.A., Laboda J.C., Costlow M.E., Photodermatol. Photoimmunol. Photomed. 13, 9-15 1997). Finally aminopsoralen derivatives are used as photosterilizing agents for hemoderivatives (F. Dall'Acqua, G.Viola, D. Vedaldi Molecular basis of psoralen photochemotherapy (Chapter 142) in CRC Handbook of Organic Photochemistry and Photobiology. W.M. Hoorspool and F. Lenci Eds.CRC Press Boca Raton USA 2004, pp. 1-17).

The chemical synthesis of angelicin and its structural derivatives has been described in the literature (U.S.A. patent 5179217; Kong LD, Tan RX, Woo AY, Cheng CH. Pharmacol Toxicol, 88(2):75-80, 2001; Mosti L, Lo Presti E, Menozzi G, Marzano C, Baccichetti F, Falcone G, Filippelli W, Piucci B. II Farmaco, 53(8-9):602-10, 1998; Sardari S, Mori Y, Horita K, Micetich RG, Nishibe S, Daneshtalab M. Bioorg Med Chem, 7(9):1933-40, 1999; Jakobs AE, Christiaens L. J Org Chem, 61(14):4842-4844, 1996; lester M, Fossa P, Menozzi G, Mosti L, Braccichetti F, Marzano C, Simonato M. II Farmaco, 50 (10): 669-678, 1995; Bisagni E. Photochem Photobiol, Review, 14(1-2):23-46, 1992)).

Known structural analogues of angelicin include for example variously substituted linear and angular furocoumarins, furocoumarin heteroanalogues, for example thiopyran-benzofuranes, acylfurocoumarins, alkylfurocoumarins and methoxyfurocoumarins. A specific example is the linear furocoumarin analogue 8-methoxypsoralen (8-MOP), which is currently used in PUVA therapy (Psoralens plus UVA radiation) to treat psoriasis and fungoid mycosis. The structural formula of 8-MOP is the following:

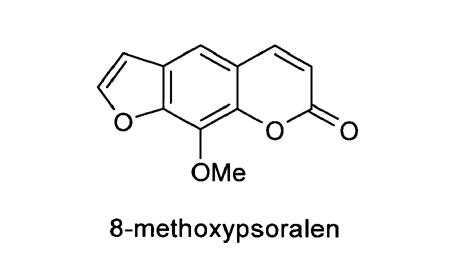

It is known that, because of their capacity to potentiate the human gamma-globin gene expression, the furocoumarins and their structural analogues can be potential pharmacological agents in the therapeutic treatment of patients suffering from beta-thalessemia.

A therapy comprising the use of molecules possessing this activity for treating patients suffering from beta-thalessemia which is more effective than known therapies could render such subjects more independent of transfusional therapy (Ikuta T, Atweh G, Boosalis V, White GL, De Fonseca S, Boosalis M, Faller DV, Perrine SP, Annals of New York Academy of Sciences, 850:87-99, 1998).

In this respect, it has been noted that very large doses of these molecules can result in undesirable toxic effects. For these reasons pharmacological strategies which enable these drugs to be used a low doses can be of great interest.

According to the present invention, furocoumarins and their linear and angular derivatives can be used in the treatment of beta-thalessemia by photoactivating said molecules with maximum effectiveness to bring them from the fundamental state to the excited state and hence render them able to increase the induction level of gamma-globin gene expression while simultaneously producing a reduced cytotoxic effect compared with that of reference drugs.

It has been surprisingly found that exposing erythroid cultures to either linear or angular furocoumarins photoactivated with UVA light results in a highly significant increase in erythroid differentiation induction and gamma-globin gene expression.

A first aspect of the present invention is therefore a drug for treating thalessemia, falciform anemia and all other forms of anemia treatable therewith as described in claim 1.

The average effective drug quantities required are decidedly less than both the pharmacologically effective concentration of angelicins in the fundamental state (reference) and those required for other drugs used in therapy such as hydroxyurea, butyrrates and 5-azacytidine.

Preferably, said furocoumarin is chosen from the group substituted furocoumarins such as acylfurocoumarin, alkyl furocoumarin, methoxyfurocoumarin and furocoumarin heteroanalogues.

A structural analogue particularly preferred for this purpose is 8-methoxypsoralen.

Moreover, as recently described (Bianchi N, Ongaro F, Chiarabelli C, Gualandi L, Mischiati C, Bergamini P, Gambari R. Biochem Pharmacol, 60:31-40, 2000;Bianchi N, Osti F, Rutigliano C, Ginanni Corradini F, Borsetti E, Tomassetti M, Mischiati C, Feriotto G and Gambari R, British Journal of Haematology, 104:258-263, 1999), a treatment combined with various modifiers of the transcription process enables the gamma-globin gene expression to be further increased.

Consequently a second aspect of the present invention is therefore a drug for treating thalessemia, falciform anemia and all other forms of anemia treatable by the drug of the invention comprising as active principle a furocoumarin in a quantity unsuitable for providing any significant therapeutic effect in the fundamental state, to be activated with UVA light after its administration and characterised by comprising, in combination with the furocoumarin as active principle, at least one further modifier of the transcription process.

According to a preferred embodiment, said further modifier of the transcription process is chosen from the group consisting of cytosine arabinoside, retinoic acid, plicamycin, hydroxyurea, guanine, guanosine triphosphate (GTP), guanosine diphosphate (GDP), guanosine monophosphate (GMP). Of these, cytosine arabinoside and retinoic acid are most preferred.

The photochemotherapy of thalessemia and of the other anemia forms treatable with furocoumarins and their derivatives activated by UVA light represents an improvement over chemotherapy alone, producing a surprisingly improved therapeutic effect.

The efficiency of the treatment according to the present invention as a method for erythroid cell differentiation and mRNA production for gamma-globin was evaluated in human cell cultures.

The results of this study are illustrated in the following examples. The data reported in the examples indicate that the efficiency of the photoactivated drug is clearly better than that of hydroxyurea, a reference drug for inducing HbF, and also better than the same drug used without photoactivation, with a considerable reduction in the concentrations required to produce the desired pharmacological effect, typically less than µM. These concentrations are ineffective inthe absence of photoactivation. Moreover, it has been verified that the cytotoxic effect encountered is negligible compared with that for hydroxyurea.

The following examples are provided by way of illustration and are not intended to limit the extent of the invention in any way.

### EXAMPLE 1

The evaluation of the biological activity of the treatment with psoralens photoactivated with UVA light was conducted by examining the capacity of these molecules to modulate the gamma-globin gene expression in the K562 human cell line, which is able to differentiate in the erythroid sense by expressing the gamma-globin genes if subjected to treatment with biological response modifiers suitable for the purpose (Lampronti I., Bianchi N Borgatti M., Fibach E.,Prus E.,Gambari R. Eur J Haematology 71 189-195 2003, Bianchi N, Ongaro F, Chiarabelli C, Gualandi L, Mischiati C, Bergamini P, Gambari R. Biochem Pharmacol, 60:31-40, 2000;Bianchi N, Osti F, Rutigliano C, Ginanni Corradini F, Borsetti E, Tomassetti M, Mischiati C, Feriotto G and Gambari R, British Journal of Haematology, 104:258-263, 1999).

The differentiation level was evaluated by analyzing the cell positivity to benzidine (Lampronti I., Bianchi N., Borgatti M., Fibach E., Prus E., Gambari R. Eur J Haematology 71 189-195 2003,).

The gamma-globin coding gene expression was evaluated by quantitative RT-PCR (reverse transcriptase PCR) (Lampronti I., Bianchi N Borgatti M., Fibach E.,Prus E.,Gambari R. Eur J Haematology. 71 189-195 2003) Some of the data obtained are reported in Table 1. As can be easily noted, treatment with different furocoumarin derivatives photoactivated with UVA light are able to induce an increase in the percentage of cells positive to benzidine (60-85% of the treated cells, compared with 5-17% of the K562 control cells treated with furocoumarin derivatives in the absence of UVA light). Treatment with 8-methoxypsoralen and in particular with trimethylangelicin photoactivated with UVA light shows the capacity to induce differentiation (measured as benzidine-positive cell increase). In the control not treated with furocoumarin derivatives, erythrolid differentiation is low (4-6%) both in the absence and in the presence of UVA light.

Erythroid differentiation in K562 cells treated with furocoumarins photoactivated with UVA light is very similar, but without toxicity, compared with that obtained with cytosin arabinoside, one of the most effective known inducers (Bianchi N, Ongaro F, Chiarabelli C, Gualandi L, Mischiati C, Bergamini P, Gambari R. Biochem Pharmacol, 60:31-40, 2000;Bianchi N, Osti F, Rutigliano C, Ginanni Corradini F, Borsetti E, Tomassetti M, Mischiati C, Feriotto G and Gambari R, British Journal of Haematology, 104:258-263, 1999).

**Table 1.**

| Compound | Concentration (µM) | ^{(a)}Erythroid differentiation without UVA (%) | ^{(a)}Erythroid differentiation with UVA (%) |
|---|---|---|---|
| Control, not treated | 0 | 4 | 6 |
| angelicin | 0.50 | 6 | 60 |
| 8-MOP | 0.25 | 17 | 65 |
| trimethylangelicin | 0.50 | 5 | 85 |
| ^{(a)} Erythroid differentiation = percentage di K562 cells positive to benzidine. The concentrations indicated for each molecule are sub-optimal doses for the purpose of achieving erythroid differentiation activation even at low dosage by photoactivation. | | | |

### EXAMPLE 2

To verify if treatment with 8-meethoxypsoralen and trimethylangelicin photoactivated with UVA light was able to stimulate mRNA production for gamma-globin in human erythroid precursors isolated from peripheral blood, the method used was that described by Fibach et al. (Fibach E. Hemoglobin, 22: 445-458, 1998; Fibach E, Burke KP, Schechter AN, Noguchi CT & Rodgers GP. Blood, 81: 1630-1635, 1993). This method comprises two steps: the cells isolated from peripheral blood of a healthy subject or affected with a hemopoietic pathology, such as falciform anemia or beta-thalessemia, are seeded in culture medium to which 10% of conditioned medium deriving from the bladder carcinoma cell line 5637 is added. The second step consists of cultivating the isolated cells in a suitable culture medium to which erythropoietin hormone, 30% of bovine fetal serum, 2-mercapto-ethanol, albumin, glutamine and desametasone are added to enable proliferation and maturing of erythroid stem cells. In this step the cells can be treated with potential HbF inducers. For example with this system it was demonstrated that hydroxyurea, a DNA synthesis inhibitor currently used in experimental beta-thalessemia therapy, is able to induce HbF production.

The results obtained using this method demonstrated an increase in gamma-globin mRNA production in cells treated with trimethylangelicin photoactivated with UVA light compared with those untreated (Table 2). The gamma-globin mRNA production level is much higher than that encountered using hydroxyurea as inducer (Fibach E. Hemoglobin, 22: 445-458, 1998; Fibach E, Burke KP, Schechter AN, Noguchi CT & Rodgers GP. Blood, 81: 1630-1635, 1993).

**Table 2**

| Compound | Concentration (µM) | ^{(a)}gamma-globulin mRNA |
|---|---|---|
| 8-MOP | 0.25 | 2.76 |
| | 1.00 | 18.49 |
| trimethylangelicin | 0.10 | 9.56 |
| a | 0.50 | 12.86 |
| ^{(a)} The values represent the ratio of mRNA content in UVA-treated cells to that of UVA-untreated cells. The RTOPCR was quantitatively determined using the following nucleotide primers and probe: gamma-globin forward primer, 5'-TGG CAA GAA.GGT GCT GAC TTC-3', gamma-globin reverse primer 5'-TCA CTC AGC TGG GCA AAG G-3', gamma-globin probe, 5'-FAM-TGG GAG ATG CCA TAA AGC ACC TGG-TAMRA-3' (FAM = 6-carboxy fluorescin, TAMRA = 6-carboxy-N,N,N',N'-tetramethylrhodamine). The quantitative RT-PCR analyses (Heid CA, Stevens J, Livak KJ & Williams PM. Genome Research, 6: 986-994, 1996; Gibson UE, Heid CA & Williams PM. Genome Research, 6: 995-1001, 1996) were carried out on material extracted from cells treated for 6 days under the conditions indicated. | | |

The results obtained show that the furocoumarins of the invention when activated with UJVA light can be advantageously used in thalessemia photochemotherapy for preparing very active drugs of low dosage, to be administered orally together with suitable additives, excipients or diluents (capsules containing 10 mg of u8-MOP with the following excipients: starch 300 mg, lactose 34 mg and 6 mg of magnesium stearate as lubricant). As an alternative to the classical photochemotherapy method, PUVA type, the drug of the invention can also be advantageously administered by the extracorporeal photochemotherapy method (photopheresis) (vials containing 200 µg of 8-MOP in 10 ml of sterile isotonic solution).

From the aforegoing it is apparent that the drug of the invention offers the following advantages:
- high pharmacological effectiveness compared with traditional drugs used for treating txt and other anemia forms,
- ability to use extremely low drug concentrations with consequent reduced undesirable effects,
- reduced toxicity.

## Claims

1. A drug for treating thalessemia, falciform anemia and all other forms of anemia treatable therewith, **characterised by** comprising as active principle a furocoumarin or a heteroanalogue thereof in a quantity unsuitable for providing any significant therapeutic effect in the fundamental state, to be activated with UVA light after its administration.

2. A drug as claimed in claim 1, **characterised in that** said quantity is less than or equal to 0.6 mg/kgv when administered orally.

3. A drug as claimed in claim 1, **characterised in that** the active principle is a linear furocoumarin (psoralen).

4. A drug as claimed in claim 1, **characterised in that** the active principle is an angular furocoumarin (angelicin).

5. A drug as claimed in claim 3 or 4, **characterised in that** the active principle is a substituted furocoumarin.

6. A drug as claimed in claim 5, **characterised in that** the active principle is an acyl furocoumarin.

7. A drug as claimed in claim 5, **characterised in that** the active principle is an alkyl furocoumarin.

8. A drug as claimed in claim 7, **characterised in that** the active principle is an alkylangelicin.

9. A drug as claimed in claim 5, **characterised in that** the active principle is a methoxyfurocoumarin.

10. A drug as claimed in claim 5, **characterised in that** the furocoumarin is 8-methoxypsoralen.

11. A drug for treating thalessemia, falciform anemia and all other forms of anemia treatable with the drug of the invention as claimed in any one of the preceding claims, **characterised by** comprising, in combination with furocoumarin or a heteroanalogue thereof, at least one further modifier of the transcription process.

12. A drug as claimed in claim 11, wherein said further modifier of the transcription process is chosen from the group consisting of cytosine arabinoside, retinoic acid, plicamycin, hydroxyurea, guanine, guanosine triphosphate (GTP), guanosine diphosphate (GDP) and guanosine monophosphate (GMP).

13. A method for activating the drug claimed in any one of the preceding claims, **characterised by** subjecting to UVA radiation a patient to whom the drug has been administered.

14. A method for activating the drug claimed in any one of claims from 1 to 12, **characterised by** subjecting to photophoresis the blood withdrawn from a patient to whom the drug has been administered.

15. A pharmaceutical composition for oral administration for the photochemotherapy of thalassemia, **characterised by** comprising at least one drug claimed in any one of claims from 1 to 12 having the following composition:
active principle: 10 mg
diluents: starch 300 mg, lactose 334 mg
lubricant: magnesium stearate 6 mg
inserted into capsules.

16. A pharmaceutical composition for photophoresis administration for the therapy of thalassemia, **characterised by** comprising at least one drug claimed in any one of claims from 1 to 12 having the following composition:
active principle: 200 µg
diluent: starch 10 ml sterile isotonoc solution.

17. A photochemotherapeutic method for treating thalassemia, **characterised by** comprising the following steps: orally administering a pharmaceutical product claimed in claims from 1 to 12; irradiating at an intensity between 10 and 13 Joule/cm² with UVA lamps; repeating the treatment sessions as follows: one per day, with three/four sessions per week for a total of 8/12 sessions.

18. An extracorporeal photochemotherapeutic method for treating thalassemia, **characterised by** comprising the following steps: collecting leukocytes separated by aferesis, photoactivating with UVA in the presence of 8-methoxypsoralen (200 ng/ml), reinfusing the photoactivated cells.
